# EUROPÄISCHE PATENTSCHRIFT

(11) **EP 0 388 831 B1**
(45) Veröffentlichungstag und Bekanntmachung des Hinweises auf die Patenterteilung: **01.09.1993**
(21) Anmeldenummer: 90105109.4
(22) Anmeldetag: 19.03.1990
(51) Int. Cl.: G01L 7/18, A61M 1/00

(54) **Anzeigevorrichtung für Gasdrücke und Verfahren zum Herstellen derselben**
Gas pressure indicating device and method for producing the same
Dispositif indicateur de pression de gaz et sa méthode de fabrication

(30) Priorität: 23.03.1989 DE 3909627
(43) Veröffentlichungstag der Anmeldung: 26.09.1990
(73) Patentinhaber: MEDINORM AKTIENGESELLSCHAFT MEDIZINTECHNISCHE PRODUKTE, D-66287 Quierschied (DE)
(72) Erfinder: Fell, Helmut, Dr. rer. nat., D-6607 St. Ingbert (DE)
(74) Vertreter: Müller, Hans, Dipl.-Ing.

(56) Entgegenhaltungen:
- DE-A- 3 725 193
- US-A- 3 541 858
- US-A- 3 838 601
- US-A- 4 079 729

## Beschreibung

Die Erfindung betrifft eine Anzeigevorrichtung für insbesondere medizintechnische Einrichtungen, mit der in einem Hohlraum wie z. B. einer Vakuumsaugflasche vorhandene Unterdrücke angezeigt werden können. Die Erfindung betrifft auch ein Verfahren zum Herstellen dieser Anzeigevorrichtung.

Eine derartige Anzeigevorrichtung besitzt ein Meßröhrchen mit einem Rohrkanal, wobei der rohrkanal teilweise mit einer Flüssigkeit gefüllt ist, und ein verschlossenes sowie ein mit dem Hohlraum verbundenes offenes Ende. Zwischen dem verschlossenen Ende des Rohrkanals und der Flüssigkeit ist eine vorbestimmte Menge eines Gases vorhanden. Je nachdem, wie hoch der in dem Hohlraum vorhandene Gasdruck ist, dehnt sich dieses zwischen dem verschlossenen Ende des Rohrkanals und der Flüssigkeit vorhandene Gas mehr oder weniger aus und verschiebt dadurch die im Röhrchen vorhandene Flüssigkeit. Die Stellung der Flüssigkeitssäule ergibt so ein Maß für den jeweils herrschenden Druck in der beispielsweise Vakuumsaugflasche.

Eine derartige Anzeigevorrichtung ist bei ihrer Verwendung als medizintechnische Einrichtung vorzugsweise ein Einwegartikel, der daher möglichst preiswert hergestellt werden soll.

### STAND DER TECHNIK

Aus der US-PS 4.079.729 ist eine Anzeigevorrichtung der eingangs genannten Art bekannt, die innerhalb eines Vakuumbehälters angeordnet ist. Die in dem Rohrkanal vorhandene Flüssigkeit wandert zwischen zwei auf dem Meßröhrchen vorhandenen Markierungen hin und her. Bei nicht mehr ausreichendem Unterdruck bewegt sich die Flüssigkeit aus dem Markierungsbereich heraus. Von Nachteil erweist sich nicht nur, daß sich mit dieser vorbekannten Anzeigevorrichtung nur qualitativ bestimmen läßt, ob der in der Flasche vorhandene Unterdruck für den vorbestimmten Zweck noch ausreicht, dagegen nicht exakt bestimmt werden kann, wie hoch der jeweilige Unterdruck in der Vakuumflasche noch ist, sondern insbesondere auch, daß sich bei relativ schnellem Druckausgleich, d. h., bei relativ schneller Bewegung der Flüssigkeitssäule, durch die in die Vakuumflasche einströmende Luft auch Luft in die Flüssigkeitssäule eintritt und zwar deshalb, weil bei schneller Bewegung der Flüssigkeitssäule ein Teil der Flüssigkeit an der Wand des Anzeigeröhrchens haften bleibt, und anschließend zu kleinen Tropfen wieder zusammenläuft, so daß die Flüssigkeitssäule geteilt wird. Eine genaue Kalibrierung dieser Anzeigevorrichtung wäre daher kaum möglich.

Eine weitere Anzeigevorrichtung der eingangs genannten Art ist aus der DE-A-37 25 193 bekannt. Diese gattungsbildende Anzeigevorrichtung ist im Gegensatz zu der aus der US-PS 4,079,729 bekannte Anzeigevorrichtung außerhalb eines Vakuumbehälters angeordnet. Um den jeweiligen Unterdruck möglichst exakt ablesen zu können, weist der Rohrkanal vom Meßröhrchen dieser Anzeigevorrichtung einen nicht konstanten Querschnitt auf. Dadurch kann eine lineare Abhängigkeit der Anzeige vom Druck erreicht werden und damit eine ebenfalls lineare Skala verwendet werden; allerdings sind die Herstellkosten für ein derartig ausgebildetes Röhrchen entsprechend hoch, was den Einsatz als Einwegartikel wirtschaftlich sehr erschwert. Ansonsten weist diese Anzeigevorrichtung alle die Nachteile auf, die auch bei der Anzeigevorrichtung gemäß der US-PS 4,079,729 vorhanden sind.

### DARSTELLUNG DER ERFINDUNG

Der Erfindung liegt die Aufgabe zugrunde, eine Anzeigevorrichtung der eingangs genannten Art anzugeben, die unter Vermeidung der aus dem Stand der Technik bekannten Vor- und Nachteile eine möglichst genaue Druckanzeige ermöglicht, die dabei aber billig herzustellen ist.

Ausgehend von dem vorbekannten Stand der Technik ist die erfindungsgemäße Anzeigevorrichtung durch die Merkmale des Patentanspruchs 1 und das entsprechende Verfahren zum Herstellen dieser Anzeigevorrichtung durch die Merkmale des Patentanspruchs 8 gegeben. Die mit der Drosselstelle bewirkte Dämpfung ermöglicht einen langsamen Druckausgleich im Rohrkanal im Bereich der Flüssigkeitssäule, so daß sich letztere nur so langsam in dem Rohrkanal verschieben kann, daß die Flüssigkeitssäule immer als durchgehende Säule erhalten bleibt. Verfälschungen des jeweils angezeigten Flüssigkeitsstandes durch Einwandern von Gasblasen in die Flüssigkeit können somit nicht auftreten. Eine Kalibrierung der Anzeigevorrichtung erweist sich damit als sinnvoll. Um zu verhindern, daß der im Inneren des Meßröhrchens vorhandene Vakuum-Druck, der dem im Inneren der Flasche vorherrschenden Vakuum entspricht, durch Gasdiffusion durch die Wandung des Meßröhrchens hindurch abgebaut wird mit der Folge, daß die Anzeigegenauigkeit des Meßröhrchens abnimmt, ist erfindungsgemäß ferner um das Meßröhrchen herum ein zusätzliches Röhrchen angeordnet, welches ein offenes Ende besitzt, mit dem es in den Hohlraum wie z. B. der Vakuumflasche gasdicht einsitzt. Sein anderes Ende ist verschlossen, so daß es kappenartig den aus dem Hohlraum herausragenden Teil des Meßröhrchens umhüllt. Um das Meßröhrchen ist damit im Bereich zwischen demselben und der Innenwandung des äußeren Röhrchens ebenfalls der in der Vakuumflasche herrschende Unterdruck vorhanden.

Die Anordnung einer Drosselstelle zwecks Herstellen eines langsamen Druckausgleichs in einem Meßröhrchen ist für sich genommen aus der US-A-3,541,858 bekannt. Hinweise dergestalt, um dieses Meßröhrchen ein weiteres Rohr anzuordnen, so daß um das Meßröhrchen im Bereich zwischen demselben und dem äußeren Röhrchen immer derselbe Unterdruck herrscht, der auch in der medizinischen Einrichtung, wie z. B. einer Vakuum-Saugflasche herrscht, an die jeweils die Anzeigevorrichtung angeschlossen ist, sind dieser Druckschrift nicht zu entnehmen.

Die Drosselstelle kann nach einer Ausführungsform der Erfindung durch eine in dem Meßröhrchen im Bereich zwischen dem offenen Ende und der Flüssigkeit einsitzendem Pfropfen gebildet werden, durch den das in dem Vakuum befindliche Gas wie insbesondere Luft nur sehr langsam hindurchströmen kann. Dieser Pfropfen kann beispielsweise aus Kunststoff hergestellt sein.

Nach einer anderen ganz wesentlichen Ausführungsform der Erfindung ist die Drosselstelle im Bereich der Flüssigsäule vorhanden. Die gewünschte Dämpfung läßt sich dann nämlich auf diese Weise sehr einfach herstellen, da sich die Flüssigkeit wesentlich einfacher "drosseln" läßt als Gas. So hat es sich unter wirtschaftlichen Gesichtspunkten als einfache Möglichkeit für die gewünschte Drosselung herausgestellt, dieselbe durch eine Verringerung des lichten Innenquerschnittes bzw. durch eine Einschnürung des Meßröhrchens herzustellen.

Die Anzeigevorrichtung kann sowohl innerhalb der Vakuumflasche als auch außerhalb derselben vorhanden sein. Sofern die Anzeigevorrichtung außerhalb der Flasche vorhanden ist, wird das in der Flasche vorhandene nutzbare Volumen nicht verringert.

Die erfindungsgemäße Anzeigevorrichtung läßt sich mit wirtschaftlich geringem Aufwand herstellen, wenn die Drosselstelle des Meßröhrchens durch eine teilweise Quetschung und dessen einer Endbereich durch eine vollständige Quetschung der Rohrwandung des Meßröhrchens hergestellt werden. Zum Herstellen des Meßröhrchens könnte dann beispielsweise zuerst die in seinem mittleren Bereich als Drosselstelle fungierende Quetschung hergestellt werden, dann von einer Seite des Röhrchens aus Flüssigkeit in dasselbe gefüllt werden, bis der Rohrkanal bis zu einer bestimmten Markierung gefüllt ist, wobei der zusammengequetschte Bereich des Röhrchens für die Flüssigkeit während des Einfüllens praktisch undurchlässig ist. Anschließend kann das Röhrchen dann in einem bestimmten Abstand oberhalb der Markierung vollständig zusammengequetscht werden und so der an dieser Stelle erforderliche einseitige Verschluß des Röhrchens hergestellt werden. So könnte auf einfache Weise immer die gleiche Gasmenge zwischen dem oberen Flüssigkeitsspiegel und dem oberen Ende des Röhrchens angeordnet werden, wodurch eine sehr einfache Kalibrierung des Meßröhrchens ermöglicht würde. Dieses Meßröhrchen würde dann anschließend in das zweite Röhrchen eingesetzt werden.

Ein anderes äußerst wirtschaftliches Herstellverfahren für die erfindungsgemäße Anzeigevorrichtung sieht folgendermaßen aus:

Zunächst werden beide Quetschungen des Meßröhrchens hergestellt, d. h. das Röhrchen wird mechanisch komplett angefertigt. Dann wird das Röhrchen kopfstehend innerhalb eines geschlossenen Behälters angeordnet und dabei in eine in diesem Behälter vorhandene Anzeigeflüssigkeit etwas eingetaucht. Durch Vermindern des im Innern des Behälters vorhandenen Druckes dehnt sich die im Röhrchen gefangene Luft aus und perlt unten aus demselben heraus. Beim anschließenden wieder Erhöhen des Druckes strömt Anzeigeflüssigkeit in das Röhrchen hinein, beispielsweise bis in Höhe der Drosselstelle. Das Röhrchen wird dann aus der Anzeigeflüssigkeit herausgenommen und der Druck im Behälter bis auf atmosphärischen Druck weiter erhöht; dadurch strömt die Anzeigeflüssigkeit durch die Drosselstelle hindurch in den darüberliegenden Röhrchenbereich. Über den jeweils gewählten Unterdruck läßt sich das von der Anzeigeflüssigkeit in dem Röhrchen eingeschlossene Gasvolumen auf einfache Weise sehr genau festlegen. Auch bei diesem Herstellverfahren wird dann anschließend dieses Meßröhrchen in das zweite Röhrchen hineingesetzt.

Wie bereits vorstehend beschrieben, kann es sinnvoll sein, wenn die Drosselstelle beidseitig von Flüssigkeit eingeschlossen ist. Dies kann bei dem vorstehend beschriebenen ersten Herstellverfahren beispielsweise dadurch verwirklicht werden, daß nach dem Verschließen des einen Endes des Röhrchens von dem anderen Ende des Röhrchens aus noch etwas Flüssigkeit in dasselbe eingefüllt wird. Diese noch einzufüllende Flüssigkeitsmenge braucht nicht exakt vorbestimmt zu werden, da der jeweils herrschende Druck an der anderen, dem verschlossenen Ende zugewandten Flüssigkeitsspiegel angezeigt wird.

Weitere Merkmale und Vorteile der Erfindung sind den in den Ansprüchen ferner aufgeführten Merkmalen sowie in dem nachfolgenden Ausführungsbeispiel angegeben.

### KURZE BESCHREIBUNG DER ZEICHNUNG

Die Erfindung wird im folgenden anhand des in der Zeichnung dargestellten Ausführungsbeispiels näher beschrieben und erläutert. Es zeigen:

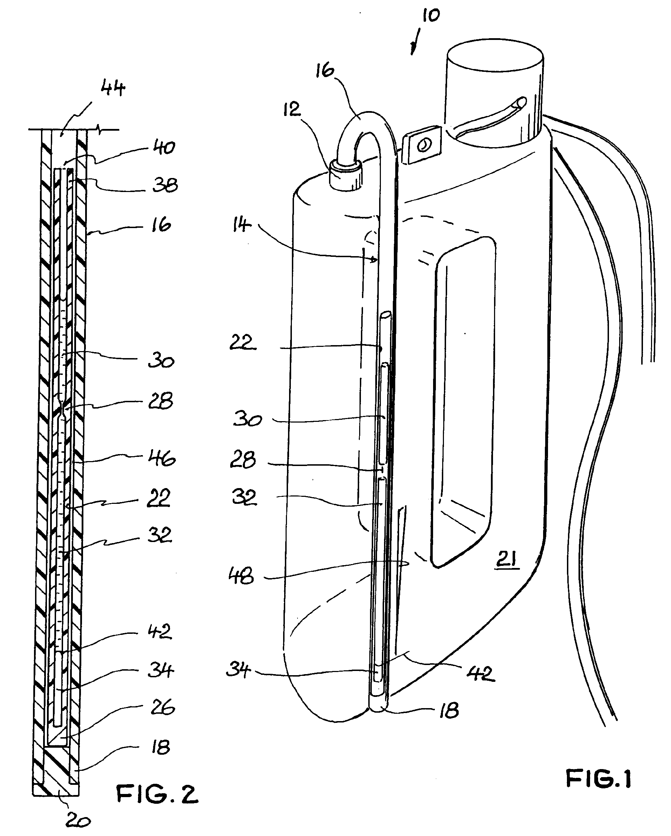

- Fig. 1: eine perspektivische schematisierte Darstellung einer Vakuumflasche mit daran befestigter erfinungsgemäßer Anzeigevorrichtung und
- Fig. 2: einen Längsschnitt durch die Anzeigevorrichtung nach Fig. 1.

### WEGE ZUR AUSFÜHRUNG DER ERFINDUNG

An einer Vakuumsaugflasche 10 ist über einen Stutzen 12 eine Anzeigevorrichtung 14 gasdicht angeschlossen.

Die Anzeigevorrichtung 14 weist einen Schlauch 16 auf, der an seinem einen - in der Zeichnung oberen - Ende offen ist und mit diesem Ende gasdicht in dem Stutzen 12 und damit in der Vakuumflasche 10 einsitzt.

Das untere Ende 18 dieses Schlauches 16 ist mittels eines Verschlußteils 20 gasdicht beispielsweise über eine Klebverbindung verschlossen.

Der Schlauch 16 ist auf der einen Außenseite - in der Zeichnung der Vorderseite 21 - in einer dort vorhandenen nutartigen Einformung eingeklebt vorhanden. Er kann dadurch seine Lage relativ zur Vakuumflasche nicht verändern.

Im Inneren dieses Schlauches 16 ist ein Röhrchen 22 vorhanden. Dieses Röhrchen ist ebenso wie der Schlauch 16 aus einem Kunststoffmaterial hergestellt. An seinem einen - in der Zeichnung unteren - Ende weist es eine Quetschstelle 26 auf, so daß es an diesem Ende vollständig verschlossen ist.

Eine weitere Quetschstelle 28 befindet sich im mittleren Bereich dieses Röhrchens 22. Diese Quetschstelle 28 verschließt das Innere des Röhrchens 22 nicht vollständig. Oberhalb dieser Quetschstelle 28 ist eine Flüssigkeitssäule 30 und unterhalb der Quetschstelle eine Flüssigkeitssäule 32 vorhanden. Die untere Flüssigkeitssäule 32 sitzt auf einem Gaspfropfen 34 auf, der innerhalb des Röhrchens 22 vorhanden und nach unten hin durch die Quetschstelle 26 begrenzt wird. Das obere Ende 38 des Röhrchens 22 weist eine Öffnung 40 auf.

Je nachdem, wie stark der Gaspfropfen 34 sich ausdehnt, ist der untere Spiegel 42 der Flüssigkeitssäule 32 unterschiedlich hoch vorhanden.

Die Anzeigevorrichtung 14 funktioniert auf folgende Weise.

Dadurch, daß der Schlauch 16 mit dem Inneren der Vakuumflasche 10 gasdicht verbunden ist, herrscht im Bereich 44, oberhalb der Öffnung 40, im Schlauch 16 der gleiche Druck wie im Inneren der Vakuumflasche 10. Der im Inneren des Schlauches 16 herrschende Druck ist auch zwischen dem Schlauch 16 und dem Röhrchen 22, d. h. im Zwischenraum 46 vorhanden. Dieser Unterdruck herrscht ferner oberhalb der Flüssigkeitssäule 30, 32. Die Flüssigkeitssäule 30, 32 wird sich folglich so innerhalb des Röhrchens 22 verschieben, daß oberhalb der Flüssigkeitssäule 30 und unterhalb der Flüssigkeitssäule 32 und damit innerhalb des Gaspfropfens 34 der gleiche Druck herrscht. Die höhenmäßige Stellung des Flüssigkeitsspiegels 42 bildet damit ein Maß für den in der Vakuumflasche herrschenden Unterdruck. Sofern sich der Flüssigkeitsspiegel 42 in seiner untersten Stellung befindet, herrscht in der Vakuumflasche 10 ein minimaler Unterdruck. Je höher die Lage des Flüssigkeitsspiegels 42 innerhalb des Röhrchens 22 ist, umso höher ist auch der in der Vakuumflasche herrschende Unterdruck. Durch an der Vakuumflasche angebrachte Markierungen 48 läßt sich so der jeweils herrschende Unterdruck feststellen.

Die Verengung 28 hat den großen Vorteil, daß die Flüssigkeitssäule 30, 32 nur sehr langsam durch die Verengung hindurchströmen kann. Bei plötzlich auftretenden, relativ starken Druckänderungen innerhalb der Vakuumflasche 10, was ansich ein entsprechend rasches Verschieben der Flüssigkeitssäule 30, 32 zur Folge hätte, wird infolge der Drosselstelle 28 ein nur langsames Verschieben der Flüssigkeitssäule 30, 32 erfolgen. Dies hat zur Folge, daß die Flüssigkeitssäule als Säule intakt vorhanden bleibt und daß die Anzeigegenauigkeit dieser Anzeigevorrichtung 14 nicht durch in die Flüssigkeitssäule eintretende Luftblasen beeinträchtigt wird. Eine Beeinträchtigung ist ferner dadurch praktisch ausgeschlossen, daß eine Gasdiffusion durch die Wandung des Röhrchens 22 hindurch praktisch ausgeschlossen ist, da der im Inneren des Röhrchens 22 herrschende Druck und der das Röhrchen 22 umgebende Druck bis auf den sehr kleinen Bereich des Gaspfropfens 34 gleich groß ist.

Sofern die Druck-Anzeigevorrichtung innerhalb der Vakuumflasche 10 angeordnet werden sollte, könnte auf den das Röhrchen 22 umgebenden Schlauch 16 verzichtet werden, da in diesem Falle das Röhrchen 22 von dem in der Vakuumflasche 10 herrschenden Druck direkt umgeben wäre.

## Patentansprüche

1. Anzeigevorrichtung (14) für insbesondere medizintechnische Einrichtungen, zur Anzeige eines in einem Hohlraum, wie z. B. einer Vakuumsaugflasche (10) vorhandenen Druckes, mit
- einem Meßröhrchen (22) mit einem Rohrkanal,
wobei der Rohrkanal
- teilweise mit einer Flüssigkeit (30, 32) gefüllt ist,
- ein verschlossenes (34) sowie ein mit dem Hohlraum verbundenes offenes Ende besitzt,
wobei in dem Rohrkanal
- zwischen dem verschlossenen Ende und der Flüssigkeit eine vorbestimmte Menge eines Gases (34) vorhanden ist,
**dadurch gekennzeichnet**, daß
- im Bereich des Rohrkanals dieses Meßröhrchens (22) eine Drosselstelle (28) derart vorhanden ist, daß die Flüssigkeit (30, 32) nur sehr langsam bei sich änderndem Hohlraum-Druck in dem Meßröhrchen sich verschieben kann,
- ein zweites Röhrchen (16) vorhanden ist, in dem das Meßröhrchen (22) vorhanden ist, und
- dieses zweite Röhrchen (16) ein offenes Ende besitzt, mit dem es in dem Hohlraum (10, 12) gasdicht einsitzt, und ein geschlossenes anderes Ende (18) besitzt, so daß es kappenartig den aus dem Hohlraum herausragenden Teil des Meßröhrchens (22) umhüllt.

2. Anzeigevorrichtung nach Anspruch 1,
**dadurch gekennzeichnet**, daß die Drosselstelle durch einen in dem Meßröhrchen im Bereich zwischen dem offenen Ende und der Flüssigkeit einsitzenden Pfropfen gebildet wird, durch den ein Gas wie insbesondere Luft nur sehr langsam hindurchströmen kann.

3. Anzeigevorrichtung nach Anspruch 2,
**dadurch gekennzeichnet**, daß der Pfropfen aus Kunststoff besteht.

4. Anzeigevorrichtung nach Anspruch 1,
**dadurch gekennzeichnet**, daß die Drosselstelle (28) im Bereich der Flüssigkeitssäule (30, 32) vorhanden ist.

5. Anzeigevorrichtung nach Anspruch 4,
**dadurch gekennzeichnet**, daß die Drosselstelle (28) durch eine Verringerung des lichten Innenquerschnittes bzw. durch eine Einschnürung des Meßröhrchens (22) gebildet wird.

6. Anzeigevorrichtung nach einem der Ansprüche 1 bis 5,
**dadurch gekennzeichnet**, daß das Meßröhrchen vollständig innerhalb des Hohlraumes vorhanden ist.

7. Anzeigevorrichtung nach einem der Ansprüche 1 bis 6,
**dadurch gekennzeichnet**, daß das Meßröhrchen (22) einen konstant großen lichten Innenquerschnitt besitzt.

8. Verfahren zum Herstellen der Anzeigevorrichtung nach einem der vorstehenden Ansprüche,
**dadurch gekennzeichnet**, daß
- das erste Röhrchen (22) in einem ersten, mittleren Bereich zusammengequetscht wird, so daß sein Rohrkanal an dieser Stelle fast vollständig verschlossen wird,
- von einer Seite des Röhrchens aus Flüssigkeit (32) in dasselbe gefüllt wird, bis der Rohrkanal bis zu einer bestimmten Markierung gefüllt ist, wobei der zusammengequetschte Bereich des Röhrchens für die Flüssigkeit praktisch undurchlässig ist,
- anschließend das Röhrchen (22) in einem bestimmten Abstand oberhalb der Markierung vollständig zusammengequetscht wird, so daß das Röhrchen an dieser Stelle vollständig verschlossen wird,
- danach das Röhrchen (22) in ein endseitig verschlossenes zweites Röhrchen (16) eingesetzt wird.

9. Verfahren nach Anspruch 8,
**dadurch gekennzeichnet**, daß nach dem Verschließen des einen Endes des ersten Röhrchens (22) von seinem anderen Ende aus etwas Flüssigkeit in das Röhrchen eingefüllt wird.

10. Verfahren zum Herstellen der Anzeigevorrichtung nach einem der Ansprüche 1 bis 7,
**dadurch gekennzeichnet**, daß
- das erste Röhrchen (22) in einem ersten, mittleren Bereich zusammengequetscht wird, so daß der Rohrkanal an dieser Stelle fast vollständig verschlossen wird, und das Röhrchen (22) an seinem einen Ende vollständig zusammengequetscht wird, so daß das Röhrchen an dieser Stelle vollständig verschlossen wird,
- das Röhrchen (22) kopfstehend in Anzeigeflüssigkeit eingetaucht wird, wobei die Anzeigeflüssigkeit mit dem Röhrchen in einem allseitig verschlossenen Behälter vorhanden,
- der Druck im Behälter vermindert und anschließend wieder verstärkt wird,
- das Röhrchen (22) aus der Flüssigkeit herausgenommen wird und der Druck im Behälter bis auf atmosphärischen Druck weiter gesteigert wird,
- danach das Röhrchen (22) in ein endseitig verschlossenes zweites Röhrchen (16) eingesetzt wird.

## Claims

1. Indicator device (14), particularly for technical medical apparatus, for indicating a pressure existing in a hollow space, such as for example a vacuum suction flask (10), comprising
- a measuring tube (22) having a tubular duct,
said tubular duct
- being partly filled with a liquid (30, 32),
- having a closed end (34) and also an open end which is connected to the hollow space, wherein in the tubular duct
- a predetermined amount of a gas (34) is present between the closed end and the liquid,
characterised in that
- in the region of the tubular duct of said measuring tube (22), a constriction (28) is present such that the liquid (30, 32) can be displaced only very slowly in the measuring tube when the pressure in the hollow space varies,
- a second tube (16) is present in which the measuring tube (22) is disposed, and
- said second tube (16) has an open end by which it is seated in a gas-tight manner in the hollow space (10, 12) and is closed at the other end (18), so that it surrounds like a cover that part of the measuring tube (22) which projects out of the hollow space.

2. Indicator device according to Claim 1, characterised in that the constriction is formed by a plug which is seated in the measuring tube in the region between the open end and the liquid and through which a gas, such as air in particular, can flow only very slowly.

3. Indicator device according to Claim 2, characterised in that the plug consists of plastics material.

4. Indicator device according to Claim 1, characterised in that the constriction (28) is present in the region of the liquid column (30, 32).

5. Indicator device according to Claim 4, characterised in that the constriction (28) is formed by a reduction of the clear inside cross-section or by a restriction of the measuring tube (22).

6. Indicator device according to one of Claims 1 to 5, characterised in that the measuring tube is disposed entirely inside the hollow space.

7. Indicator device according to one of Claims 1 to 6, characterised in that the measuring tube (22) has a clear inside cross-section of constant size.

8. Method of manufacturing the indicator device according to one of the preceding claims, characterised in that
- the first tube (22) is pinched together in a first, central region so that its tubular duct is almost completely closed at that point,
- liquid (32) is filled into the tube from one end until the tubular duct is filled to a determined mark, the region of the tube that has been pinched together being practically impermeable to the liquid,
- the tube (22) is thereupon completely pinched together at a determined distance above the mark, so that at that point the tube is completely closed,
- whereupon the tube (22) is inserted into a second tube (16) closed at its end.

9. Method according to Claim 8, characterised in that after the one end of the first tube (22) has been closed some liquid is filled into the tube from its other end.

10. Method of manufacturing the indicator device according to one of Claims 1 to 7, characterised in that
- the first tube (22) is pinched together in a first, central region so that the tubular duct is almost completely closed at that point, and the tube (22) is completely pinched together at its one end so that at that point the tube is completely closed,
- the tube (22) is immersed upside-down in indicator liquid, the indicator liquid being contained together with the tube in a container closed on all sides,
- the pressure in the container is reduced and then increased again,
- the tube (22) is taken out of the liquid and the pressure in the container is further increased to atmospheric pressure,
- whereupon the tube (22) is inserted into a second tube (16) closed at its end.

## Revendications

1. Dispositif d'indication (14) en particulier pour équipements techniques médicaux, destiné à l'indication d'une pression régnant dans une enceinte telle que par exemple une bouteille d'aspiration sous vide (10), avec
- un tube de mesure (22) à canal tubulaire,
le canal tubulaire
- étant partiellement rempli d'un liquide (30, 32),
- possédant une extrémité fermée (34) ainsi qu'une extrémité ouverte reliée à l'enceinte, tandis que dans le canal tubulaire,
- une quantité prédéterminée d'un gaz (34) est située entre le liquide et l'extrémité fermée,
caractérisé en ce que
- dans la zone du canal tubulaire de ce tube de mesure (22), un étranglement (28) est disposé de telle sorte que le liquide (30, 32) ne peut se déplacer que très lentement dans le tube de mesure lorsque la pression dans l'enceinte se modifie,
- il existe un second tube (16) dans lequel est placé le tube de mesure (22), et
- ce second tube (16) possède une extrémité ouverte, qui est placée hermétiquement dans l'enceinte (10,12), et une autre extrémité (18) fermée, de sorte qu'il entoure à la manière d'une coiffe la partie du tube de mesure (22) qui déborde de l'enceinte.

2. Dispositif d'indication selon la revendication 1, caractérisé en ce que l'étranglement est formé par un bouchon placé dans le tube de mesure dans la zone située entre l'extrémité ouverte et le liquide, un gaz tel qu'en particulier de l'air ne pouvant traverser ce bouchon que très lentement.

3. Dispositif d'indication selon la revendication 2, caractérisé en ce que le bouchon est constitué de matière synthétique.

4. Dispositif d'indication selon la revendication 1, caractérisé en ce que l'étranglement (28) est situé dans la partie remplie par la colonne de liquide (30, 32).

5. Dispositif d'indication selon la revendication 4, caractérisé en ce que l'étranglement (28) est formé par une réduction de la petite section transversale intérieure ou par un pinçage du tube de mesure (22).

6. Dispositif d'indication selon l'une des revendications 1 à 5, caractérisé en ce que le tube de mesure est situé entièrement à l'intérieur de l'enceinte.

7. Dispositif d'indication selon l'une des revendications 1 à 6, caractérisé en ce que le tube de mesure (22) présente une petite section transversale intérieure de valeur constante.

8. Procédé de fabrication du dispositif d'indication selon l'une des revendications précédentes, caractérisé en ce que
- le premier tube (22) est écrasé dans une première partie médiane de telle sorte que son canal tubulaire soit presque complètement fermé en cet emplacement,
- le tube est rempli de liquide (32) par un côté, jusqu'à ce que le canal tubulaire soit rempli jusqu'à une certaine marque, la partie écrasée du tube étant pratiquement imperméable au liquide,
- le tube (22) est ensuite entièrement écrasé à une certaine distance au-dessus de la marque, de sorte qu'en cet endroit le tube soit complètement fermé,
- le tube (22) est ensuite inséré dans un second tube (16) refermé à une de ses extrémités.

9. Procédé selon la revendication 8, caractérisé en ce qu'après la fermeture d'une extrémité du premier tube (22), une certaine quantité de liquide est versée dans le tube par son autre extrémité.

10. Procédé de fabrication du dispositif d'indication selon l'une des revendications 1 à 7, caractérisé en ce que
- le premier tube (22) est écrasé dans une première zone médiane de telle sorte que le canal tubulaire soit pratiquement complètement fermé en cet emplacement, et le tube (22) est complètement écrasé à une de ses extrémités de telle sorte qu'à cet emplacement le tube soit complètement fermé,
- le tube (22) est plongé tête en bas dans un liquide indicateur, le liquide indicateur étant situé avec le tube dans un récipient complètement fermé,
- la pression dans le récipient est réduite et ensuite à nouveau rehaussée,
- le tube (22) est retiré du liquide et la pression dans le récipient est à nouveau rehaussée jusqu'à la pression atmosphérique,
- le tube (22) est ensuite enfoncé dans un second tube (16) refermé à une de ses extrémités.
